# EUROPEAN PATENT APPLICATION

(11) **EP 4 231 237 A1**
(43) Date of publication of application: **23.08.2023**
(21) Application number: 22157976.6
(22) Date of filing: 22.02.2022
(51) Int. Cl.: G06T 7/254, A61L 2/00, A61L 2/10, A61L 2/24, A61L 9/20

(54) **CONTROL ENGINE, SYSTEM AND METHOD FOR CONTROLLING ONE OR MORE UV-C LIGHT SOURCES**

(71) Applicant: BrainLit AB, 223 63 Lund (SE)
(72) Inventor: Singvall, Jakob, 237 34 Bjärred (SE); Wingren, Tord, 234 39 Lomma (SE); Lindoff, Bengt, 237 35 Bjärred (SE); Hultin, Olof, 216 11 Limhamn (SE)
(74) Representative: AWA Sweden AB

(57) **Abstract**

The present invention relates to a control engine (20) configured to control one or more UV-C light sources (110) configured to emit UV-C light in a space (1). The control engine (20) comprising circuitry (21) configured to execute: a reference image acquiring function (24) configured to construct a reference image using reference radar sensor signals received, at a first time-instance, from a plurality of radar sensors (120) arranged in the space (1); a probing radar acquiring function (25) configured to construct a probing image using probing radar sensor signals received, at a second time-instance at which the one or more UV-C light sources (110) are inactive, from the plurality of radar sensors (120), wherein the second time-instance is after the first time-instance; and a control function (26). The control function (26) is configured to obtain a difference metric between the probing image and the reference image. Upon the difference metric being smaller than a threshold, the control function (26) is configured to activate the one or more UV-C light sources (110). Upon the difference metric being equal to or above the threshold, the control function (26) is configured to issue an alarm event.

## Description

### Technical field

The present invention relates to control of one or more UV-C light sources for decontamination of areas in a space. Especially, the present invention relates to determining when it is safe activating the one or more UV-C light sources.

### Background

Light is crucial for the well-being of humans and animals. With the technical evolution of artificial light, much driven by the development of light emitting diodes, improved light sources are now in the hands of the light designers. The light sources allow, for example, for mimicking of daylight in an indoor environment, but also offer new possibilities to create unique lighting within an environment, also with wavelengths outside for the human visual light spectrum.

For instance, short-wavelength ultraviolet (ultraviolet C or UV-C) light, typically within 100-300 nm, can be used for disinfection since UV-C can kill or inactivate microorganisms by destroying nucleic acids and disrupting their DNA, leaving them unable to perform vital cellular functions. UV-C light system may hence be implemented for disinfection purposes, or one can incorporate UV-C light sources in standard light system and by that also include disinfection capability in such a system. However, the safety of humans or animals must be considered when designing such lighting solutions and therefore there need to be safety mechanism making sure no humans or animals are harmed during the disinfection procedure. This is especially important upon applying UV-C disinfection in subareas or zones in large open rooms such as office landscapes.

### Summary of the invention

It is an object to, at least partly, mitigate, alleviate, or eliminate one or more of the above-identified deficiencies in the art and disadvantages singly or in any combination and solve at least the above-mentioned problem. According to a first aspect control engine configured to control one or more UV-C light sources configured to emit UV-C light in a space is provided. The control engine comprising circuitry configured to execute: a reference image acquiring function configured to construct a reference image using reference radar sensor signals received, at a first time-instance, from a plurality of radar sensors arranged in the space; a probing radar acquiring function configured to construct a probing image using probing radar sensor signals received, at a second time-instance at which the one or more UV-C light sources are inactive, from the plurality of radar sensors, wherein the second time-instance is after the first time-instance; and a control function. The control function is configured to: obtain a difference metric between the probing image and the reference image. The control function is further configured to upon the difference metric being smaller than a threshold, activate the one or more UV-C light sources, or upon the difference metric being equal to or above the threshold, issue an alarm event.

The present invention provides for minimizing the risk of for humans or animals being exposed for UV-C light during UV-C light disinfection of a space or portion of a space. The space or portion of a space being an office landscape, a lounge, a lobby, a hallway, a passage, etc. This by comparing changes in the space between a commissioning/calibration stage and a time when disinfection by UV-C light emission is wanted. The changes are monitored using radar sensors arranged in the space. At a commissioning/calibration stage (the first time-instance), when the space covered by the one or more UV-C light sources is empty from animals and humans etc., a reference image of the space is constructed. Once a disinfection of the space or portion of the space is wanted, a check using the radar sensors for constructing a probing image is made. The probing image is compared with the reference image for determining a difference metric between the probing image and the reference image. Based on the difference metric a decision whether it is safe or not to activate the one or more UV-C light sources can be made. If difference metric is equal to or larger than a threshold an indication for risk for harmful UV-C light reflections in the space or portion of the space is detected and an alarm event is triggered. If the difference metric is below the threshold the one or more UV-C light sources can be activated. The present invention provides for a robust detection whether activation of one or more UV-C light sources can be made or not.

The circuitry of the control engine may further be configured to execute a transformation function. The transformation function is configured to transform a radar image constructed from radar sensor signals to an UV-C light reflection image, wherein the transformation is dependent on a wavelength of EM-radiation emitted from the radar sensors and a wavelength of EM-radiation emitted from the one or more UV- C light sources. By this the reference image acquiring function may be configured to construct the reference image as a reference UV-C light reflection image transformed from a reference radar image constructed from the reference radar sensor signals. Further, the probing image acquiring function may be configured to construct the probing image as a probing UV-C light reflection image transformed from a probing radar image constructed from the probing radar sensor signals. This allows for taking into account that radar signals and UV-C light behave differently, e.g. they reflect differently from a same material. This due to different wavelengths of the EM-radiation. Hence, taking knowledge of radar wavelength and UV-C wavelength into account, a more accurate prediction of UV-C refection image can be made.

The circuitry of the control engine may further be configured to execute a material property determination function. The material property determination function is configured to determine material properties of surfaces in the space from radar sensor signals received from the plurality of radar sensors arranged in the space. By this the transformation performed by the transformation function may further be made dependent on the material properties of the surfaces in the space.

The circuitry of the control engine may further be configured to execute a correlation function. The correlation function is configured to: construct a reference radar image from the reference radar sensor signals; activate the one or more UV-C light sources; construct a reference UV-C light reflection image from reference UV-C sensor signals received, at the first time-instance, from one or more UV-C sensors arranged in the space; and determine spatial correlation information from a spatial correlation between the reference UV-C light reflection image and the reference radar image. Using this the probing image acquiring function may be configured to construct the probing image from the probing radar sensor signals and the spatial correlation information.

The circuitry of the control engine may further be configured to execute an object detection function. The object detection function is configured to: at the first time-instance, acquire a reference picture from a camera arranged in the space; at the second time-instance, acquire a probing picture from the camera; perform an object detection and identification based on differences between the reference picture and the probing picture; and determine if the object detection and identification is indicative of that a none-UV-C reflective object is responsible for the differences between the reference picture and the probing picture and if so, clear the alarm event. Hence, a camera can be used for determining the object creating the potential harmful UV-C light reflections. Hence, by using the object detection function it can be detected which kind of object is responsible for reflection of radar signal confirming or not confirming the risk of harmful UV-C light reflections. This may minimize the number of false alarms. This may provide a balance between minimizing the risk for exposing a human/animal for UV-C light and providing disinfection using the one or more UV-C light sources.

According to a second aspect a UV-C light system is provided. The UV-C light system comprising one or more UV-C light sources configured to emit UV-C light in a space; a plurality of radar sensors arranged in the space; and a control engine according to the first aspect.

The UV-C light system may further comprise one or more UV-C sensors arranged in the space needed for the correlation function to be executed.

The UV-C light system may further comprise a camera arranged in the space needed for the object detection function to be executed.

The above-mentioned features of the first aspect, when applicable, apply to this second aspect as well. In order to avoid undue repetition, reference is made to the above.

According to a third aspect a method for controlling one or more UV-C light sources configured to emit UV-C light in a space is provided. The method comprising: constructing a reference image using reference radar sensor signals received, at a first time-instance, from a plurality of radar sensors arranged in the space; constructing a probing image using probing radar sensor signals received, at a second time-instance at which the one or more UV-C light sources are inactive, from the plurality of radar sensors, wherein the second time-instance is after the first time-instance; obtaining a difference metric between the probing image and the reference image; upon the difference metric being smaller than a threshold, activating the one or more UV-C light sources; and upon the difference metric being equal to or above the threshold, issuing an alarm event.

The method may further comprise transforming a radar image constructed from radar sensor signals to an UV-C light reflection image. The transformation may be dependent on a wavelength of EM-radiation emitted from the radar sensors and a wavelength of EM-radiation emitted from the one or more UV- C light sources. The reference image may be constructed as a reference UV-C light reflection image transformed from a reference radar image constructed from the reference radar sensor signals. The probing image may be constructed as a probing UV-C light reflection image transformed from a probing radar image constructed from the probing radar sensor signals.

The method may further comprise determining material properties of surfaces in the space from radar sensor signals received from the plurality of radar sensors. The transformation may be dependent on the material properties of the surfaces in the space.

The method may further comprise: constructing a reference radar image from the reference radar sensor signals; activating the one or more UV-C light sources; constructing a reference UV-C light reflection image from reference UV-C sensor signals received, at the first time-instance, from one or more UV-C sensors arranged in the space; and determining spatial correlation information from a spatial correlation between the reference UV-C light reflection image and the reference radar image. The probing image may be constructed from the probing radar sensor signals and the spatial correlation information.

The method may further comprise: at the first time-instance, acquiring a reference picture from a camera arranged in the space; at the second time-instance, acquiring a probing picture from the camera; performing an object detection and identification based on differences between the reference picture and the probing picture; and determining if the object detection and identification is indicative of that a none-UV-C reflective object is responsible for the differences between the reference picture and the probing picture. If so, clearing the alarm event.

The above-mentioned features of the first and second aspects, when applicable, apply to this second aspect as well. In order to avoid undue repetition, reference is made to the above.

According to a fourth aspect a non-transitory computer-readable storage medium is provided. The non-transitory computer-readable storage medium having stored thereon instructions for implementing the method according to the third aspect, when executed on a device having processing capabilities.

A further scope of applicability will become apparent from the detailed description given below. However, it should be understood that the detailed description and specific examples are given by way of illustration only.

It is to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief description of the drawings

The above and other aspects will now be described in more detail, with reference to appended figures. The figures should not be considered limiting; instead, they are used for explaining and understanding.

As illustrated in the figures, the sizes of layers and regions may be exaggerated for illustrative purposes and, thus, are provided to illustrate the general structures. Like reference numerals refer to like elements throughout.
Fig. 1 illustrates a UV-C light system upon constructing a reference image.
Fig. 2 illustrates a UV-C light system upon constructing a probing image.
Fig. 3 illustrates a UV-C light system upon activating UV-C light sources of the UV-C light system.
Fig. 4 is a block diagram of a method for controlling one or more UV-C light sources configured to emit UV-C light in a space

### Detailed description

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and to fully convey the scope of the invention to the skilled person.

The basic concept of the present invention is to pro-actively detect harmful UV-C light reflections, and by that minimizing the risk for humans or animals being exposed for UV-C light.

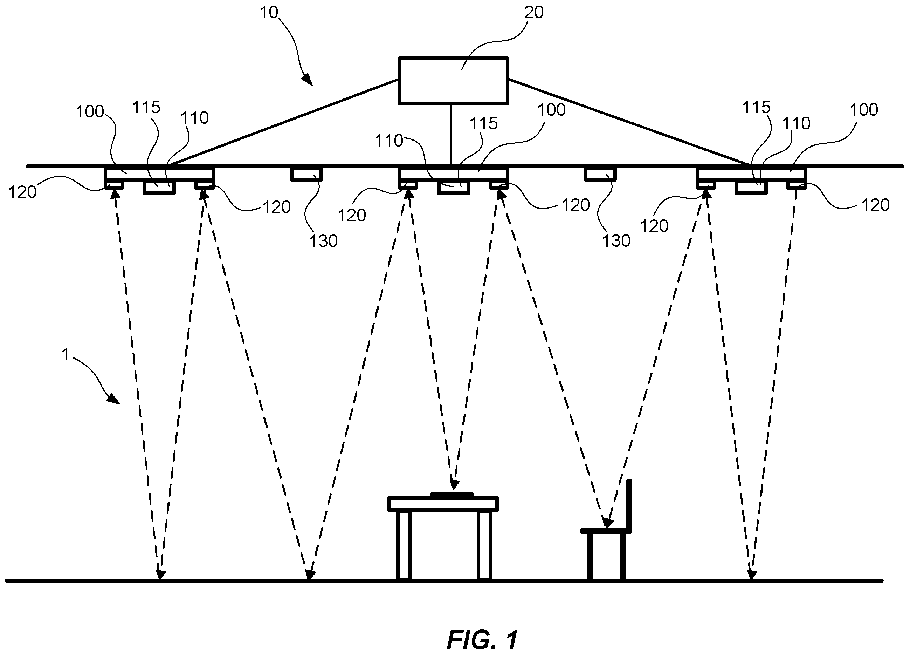

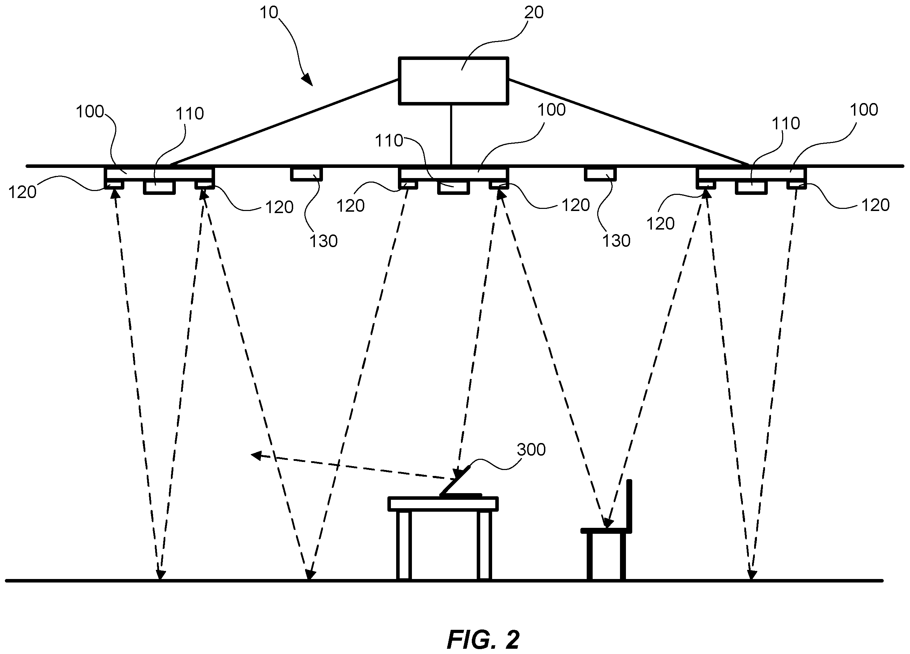

Figs 1 and 2 illustrates a UV-C light system 10 as seen at two different time-instances. The UV-C light system 10 is arranged in a space 1. The space 1 may be part of a large room of a building. That is, the space may be a subarea or zone in a large open room such as an office landscape, a lounge, a lobby, a hallway, a passage, etc. The UV-C light system 10 comprises one or more UV-C light sources 110, a plurality of radar sensors 120 and a control engine 20. The UV-C light system 10 may be a standalone system dedicated to emitting UV-C light for disinfection. However, the UV-C light system 10 may alternatively be part of a light system supporting both standard light sources emitting light in the visible wavelength range and UV-C light sources. In the present disclosure the light system will be discussed as a standalone UV-C light system, however it is to be understood that a light system supporting both standard light sources and UV-C light sources may equally be used. Hence, the standard light sources emitting light in the visible wavelength range will not be discussed in this disclosure.

In Fig.1 the UV-C light system 10 is illustrated at a first time-instance upon which a reference image is constructed by means of radar sensor signals from the radar sensors of the UV-C light system 10. In Fig. 2 the UV-C light system 10 is illustrated at a second time-instance upon which there is a need for disinfection of the space 1. At this second time-instance a probing image is constructed by means of radar sensor signals from the radar sensors of the UV-C light system 10. The construction of the reference image and the probing image will be discussed in more detail below. By comparing information of the probing image with information of the reference image a decision on whether it is safe to active the one or more UV-C light sources 110 can be made. This will also be discussed in more detail below.

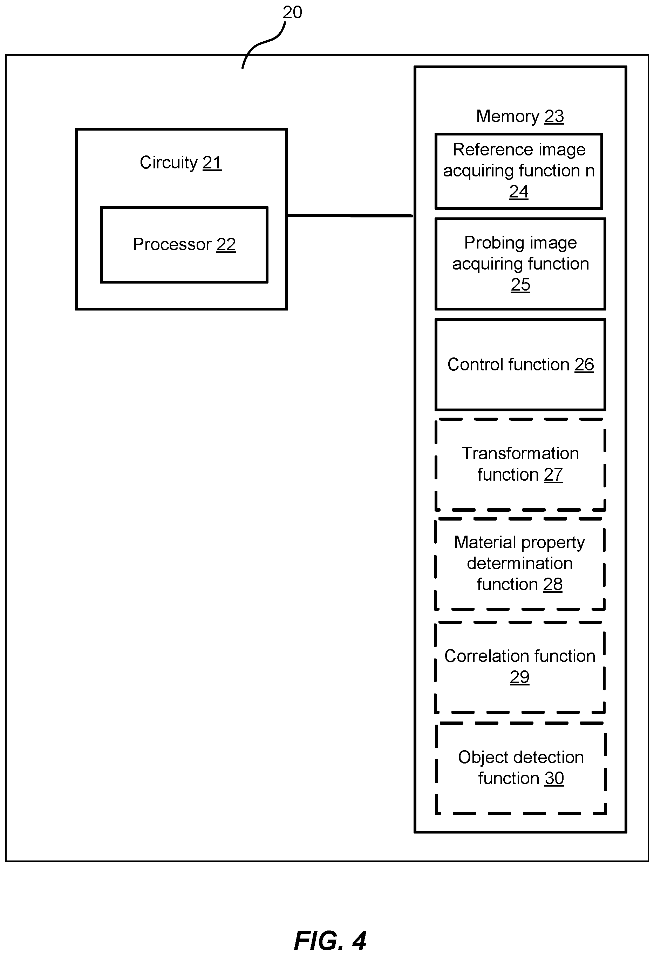

The control engine 20 is communicably connected to the one or more UV-C light sources 110 and to the plurality of radar sensors 120. Such communicably connection may be by a wire connection or as a wireless connection. The control engine 20 is configured to control the one or more UV-C light sources 110. The control engine 20 is further configured to receive radar sensor signals from the plurality of radar sensors 120. The control engine will be discussed in more detail below in connection with Fig. 4.

A subset of the one or more UV-C light sources 110 and a subset of the plurality of radar sensors 120 may be arranged in an arrangement 100. Each such arrangement may comprise one or more UV-C light sources 110 and one or more radar sensors 120. Each arrangement 100 may be communicably connected to the control engine 20.

The radar sensors 120 are configured to transmit radar pulses and to receive radar reflections. The radar pulses and radar reflections are indicated as dashed lines in Figs. 1 and 2. The radar sensors 120 are further configured to transmit radar sensor signals to the control engine 20. The radar sensor signals may be used for constructing a radar image of the space 1. The radar sensors 120 may be configured to emit and receive mmWave (frequency range 30-300 GHz) radar pulses and radar reflections. Alternatively, or in combination the radar sensors 120 may be configured to emit and receive UWB radar pulses (frequency range 3-11 GHz) and radar reflections. The radar sensors 120 may comprise an antenna array. The antenna array may be controlled in a beamforming manner so that transmitted radar pulses have a spatial direction. The spatial direction may be tagged to the transmitted radar pulses. Hence the received radar reflection may be stored with spatial direction tags. This will enable the construction of a more detailed radar image.

As discussed above the control engine 20 is configured to control the one or more UV-C light sources 110. In order to control enabling and disabling of the one or more UV-C light sources 110 information of UV-C reflections in the space is favorable. By gathering information of UV-C reflections in the space 1 it may be safeguarded that no harm on humans or animals is made due to reflections of UV-C light onto the humans or animals. For this purpose, the control engine 20 is configured to construct a probing image at a time-instance upon which activation of the one or more UV-C light sources 110 is wanted. This time-instance will be referred to as a second time-instance. An example of a situation upon the probing image is constructed is illustrated in Fig. 2. Information from such a probing image is then to be compared with information from a reference image. The reference image having been constructed at a first time-instance. The first time-instance being before the second time instance. An example of a situation upon the reference image is constructed is illustrated in Fig. 1. Based on the comparison a decision can be made whether it is safe to activate the one or more UV-C light sources 110. Especially, after such comparison it may be safeguarded that the risk for unwanted UV-C reflections in the space 1 is limited.

The reference image is constructed at the control engine 20. The reference image is constructed at the first time-instance. The first time-instance may be upon commissioning the UV-C light system 10. Alternatively, or in combination, the first time-instance may be at regular time intervals for calibration purposes. Constructing the reference image comprises transmitting radar pulses from the plurality of radar sensor 120 and receiving radar pulse reflections at the radar sensors 120. The radar pulse reflections are then used to construct a reference radar image. The reference radar image may be seen as the reference image.

The probing image is constructed at the control engine 20. The probing image is constructed at the second time-instance. The second time-instance is a time-instance upon disinfection using the one or more UV-C light source 110 is wanted. Constructing the probing image comprises transmitting radar pulses from the plurality of radar sensor 120 and receiving radar pulse reflections at the radar sensors 120. The radar pulse reflections are then used to construct a probing radar image. The probing radar image may be seen as the probing image.

For some embodiments an adjustment may be made in order to take into account that radar reflections will behave differently than UV-C light reflections in the space 1. Hence, a transformation from the mmWave or UWB constructed reference/probing radar image to a reference/probing UV-C light reflection image can be made. Performing such a transformation it is to be considered that UV-C light is in the 100-300 nm wavelength range while radar pulses are in the mmWave or UWB range. Such difference in wavelength will make reflection to behave differently. Especially, for a given material the reflection coefficient will differ for different wavelengths. Hence, by knowledge of which material is present at surfaces in the space 1, a transform from the reference/probing radar image to a reference/probing UV-C light reflection image can be made. Information on which material is present at different surfaces in the space 1 may be found by having knowledge of properties of the transmitted radar pulses, the received radar reflections, and a time response profile. By having knowledge of the material at a specific surface in the space, data on reflection coefficient for different wavelengths may be gathered, e.g., from a look-up table, for such specific surface. Having knowledge of the reflection coefficients for different wavelengths at specific surfaces, a transform of the reference/probing radar image to a reference/probing UV-C light reflection image can be made. The reference/probing UV-C light reflection image may thereafter be considered as the reference/probing image.

As an example, reference radar sensor signals, e.g., comprising radar signal reflection energy (measured e.g., in dBm) and time-delay profile, are stored for respective radar sensor at the first time-instance. This data constitutes a reference radar reflection-matrix M^{ref}_{radar}. The reference radar reflection-matrix M^{ref}_{radar} may then be transformed via a transformation matrix C to the reference UV-C light reflection image M^{ref}_{UV-C} =C* M^{ref}_{radar}. The M^{ref}_{UV-c} may thereafter be considered as the reference image. Then at the second time-instance, probing radar sensor signals are stored for respective radar sensor. This data constitutes a probing radar reflection-matrix M^{prob}_{radar}. The probing radar reflection-matrix M^{prob}_{radar} may then be transformed via a transformation matrix C' to the probing UV-C light reflection image M^{prob}_{UV-C} =C'* M^{prob}_{radar}. The M^{prob}_{UV-C} may thereafter be considered as the probing image. In a typical implementation C and C' are the same. However, C and C' may be different. For example, C' may take uncertainties, e.g., statistical variations in the probing radar reflection-matrix M^{prob}_{radar} into account.

Upon the probing image have been constructed it can be compared with the reference image. By comparing information of the probing image and the reference image a difference metric is obtained. The difference metric comprises differences between the probing image and the reference image.

In case there is no or small differences in the difference metric, i.e., the difference metric is below a threshold, the one or more UV-C light sources 110 can be activated. This since such a small difference metric is proof for that no humans or animals is in the space and the no harmful UV-C reflections is present.

A difference metric being equal to or above the threshold is an indication of that activation of the one or more UV-C light sources 110 may be harmful. For example, less reflected energy received in the probing image than in the reference image or reflection energy from the received radar reflections in the probing image being distributed significantly differently as compared to the reference image, indicates something in the space 1 have changed and hence there is a risk for abnormal UV reflection outside the expected disinfection area. This is illustrated in Fig. 2 by that a cover of an open laptop 300 is reflecting the radar pulses differently and hence the difference matrix will be above the threshold. With reference to M^{prob}_{UV-C} and M^{ref}_{UV-C} discussed above the difference metric may be expressed as a difference matrix D, where D = | | M^{prob}_{UV-C} - M^{ref}_{UV-C} | |. Hence, D being a matrix norm measuring distances between two matrices. In some embodiments the transformation matrix C/C', may be skipped and the difference matrix is obtained as D = | | M^{prob}_{radar} - M^{ref}_{radar} | | .

At the second time-instance at which time there is a need for disinfection using the one or more UV-C light sources 110 the radar sensors 120 may be used in order to check whether a human or animal is present in the space 1. Upon it is detected that a human or animal is present in the space 1 activation of the one or more UV-C light sources 110 is to be disabled. Moreover, upon it is detected that a human or animal is present in the space 1 there is no need for performing the construction of the probing image. As an alternative or complement to check whether a human or animal is present in the space 1 using the radar sensors 120, a camera 130 may be used to make sure no human or animal is within the space 1 that should be disinfected.

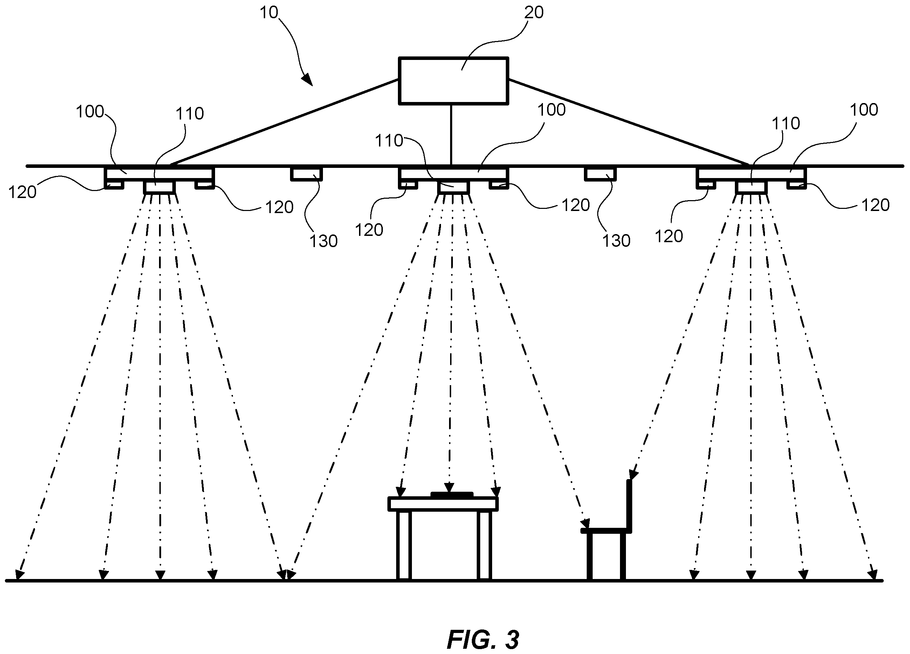

In Fig. 3 activation of the one or more UV-C light sources 110 is illustrated. The UV-C light is illustrated as dash-dotted lines. Before activation of the one or more UV-C light sources 110 a check in line with the discussion above in connection with Fig. 2 was performed. In the check it was found that the difference metric was below the threshold. Hence, activation of the one or more UV-C light sources were made possible.

In connection with Fig 4 the control engine 20 will be discussed in more detail. The control engine 20 may be embodied in a specific device, e.g., a server. However, as readily understood by the skilled person the control engine 20 may be distributed over a plurality of devices. The control engine 20 comprises circuitry 21. The circuitry 21 may include a processor 22, such as a central processing unit (CPU), microcontroller, or microprocessor. The circuitry 21 is configured to execute program code stored in a memory 23, in order to carry out functions and operations of the control engine 20.

The memory 23 may be one or more of a buffer, a flash memory, a hard drive, a removable medium, a volatile memory, a non-volatile memory, a random access memory (RAM), or another suitable device. In a typical arrangement, the memory 23 may include a non-volatile memory for long term data storage and a volatile memory that functions as system memory for the control engine 20. The memory 23 may exchange data with the circuitry 21 over a data bus. Accompanying control lines and an address bus between the memory 23 and the circuitry 21 also may be present.

Functions and operations of the control engine 20 may be embodied in the form of executable logic routines (e.g., lines of code, software programs, etc.) that are stored on a non-transitory computer readable medium (e.g., the memory 23) of the control engine 20 and are executed by the processor 21. Furthermore, the functions and operations of the control engine 20 may be a stand-alone software application or form a part of a software application that carries out additional tasks related to the control engine 20. The described functions and operations may be considered as part of a method that the control engine 20 is configured to carry out. Also, while the described functions and operations may be implemented in software, such functionality may as well be carried out via dedicated hardware or firmware, or some combination of hardware, firmware and/or software.

The control engine 20 is configured to execute a reference image acquiring function 24. The reference image acquiring function 24 is configured to construct a reference image using reference radar sensor signals received, at the first time-instance, from the plurality of radar sensors 120. The construction of the reference image is discussed in greater detail above and reference is made to that discussion.

The control engine 20 is further configured to execute a probing radar acquiring function 25. The probing radar acquiring function 25 is configured to construct a probing image using probing radar sensor signals received, at the second time-instance, from the plurality of radar sensors 120. At the second time-instance the one or more UV-C light sources 110 are inactive. The second time-instance is after the first time-instance. The construction of the probing image is discussed in greater detail above and reference is made to that discussion.

The control engine 20 is further configured to execute a control function 26. The control function 26 is configured to obtain a difference metric between the probing image and the reference image. How to obtain the difference metric is discussed in greater detail above and reference is made to that discussion. Upon the difference metric being smaller than a threshold, the control function 26 is configured to activate the one or more UV-C light sources 110. Upon the difference metric being equal to or above the threshold, the control function 26 is configured to issue an alarm event. The alarm event may be sent to an operator of the UV-C light system 10. The issuance of the alarm event may also trigger that it will be prohibited for the control function 26 to activate the one or more UV-C light sources 110.

The control engine 20 may further be configured to execute a transformation function 27. The transformation function 27 is configured to transform a radar image constructed from radar sensor signals to an UV-C light reflection image. The transformation is dependent on a wavelength of electromagnetic, EM, radiation emitted from the radar sensors 120 and a wavelength of EM-radiation emitted from the one or more UV- C light sources 110. The reference image acquiring function 24 may be configured to construct the reference image as a reference UV-C light reflection image transformed from a reference radar image constructed from the reference radar sensor signals. The probing image acquiring function 25 may be configured to construct the probing image as a probing UV-C light reflection image transformed from a probing radar image constructed from the probing radar sensor signals. The transformation from "radar space" to "UV-C light space" is discussed in in greater detail above and reference is made to that discussion.

The control engine 20 may further be configured to execute a material property determination function 28. The material property determination function 28 is configured to determine material properties of surfaces in the space 1. The material properties of surfaces in the space 1 can be determined from radar sensor signals received from the plurality of radar sensors 120. The radar sensor signals used for determining the material properties of surfaces in the space 1 may be the reference radar sensor signals but may also be made using other radar sensor signals. The transformation performed by the transformation function 27 may further be dependent on the material properties of the surfaces in the space 1. Determination of material properties of surfaces in the space 1 from radar sensor signals is discussed in in greater detail above and reference is made to that discussion.

The UV-C light system 10 may further comprise UV sensors 115. A UV sensor 115 may be a dedicated UV-C sensor or a UV-C sensitive camera. The UV-sensors 115 are placed at various places in the space 1. For example, each arrangement 100 may comprise one or more UV-sensors 115. The UV-sensors 115 may be forming part of the UV-C light source(s). The UV sensors 115 may be used to measure UV reflections within the space 1. For example, the UV sensors 115 may be used to measure UV reflections at the first time-instance. Such measure of UV reflections may be spatially correlated to the reference radar image and a mapping between radar reflections and UV reflections can be made. Such mapping may be used in order to determine the transformation matrix C/C'.

The control engine 20 may further be configured to execute a correlation function 29. The correlation function 29 is configured to construct a reference radar image from the reference radar sensor signals. The correlation function 29 is further configured to activate the one or more UV-C light sources and construct a reference UV-C light reflection image from reference UV-C sensor signals received from one or more UV-C sensors 115 arranged in the space 1. The correlation function 29 is further configured to determine spatial correlation information from a spatial correlation between the reference UV-C light reflection image and the reference radar image. The probing image acquiring function 25 may then be configured to construct the probing image from the probing radar sensor signals and the spatial correlation information.

The control engine 20 may further be configured to execute an object detection function 30. The object detection function 30 is configured to, at the first time-instance, acquire a reference picture from a camera 130 arranged in the space 1, and, at the second time-instance, acquire a probing picture from the camera 130. The object detection function 30 is further configured to perform an object detection and identification based on differences between the reference picture and the probing picture. The object detection function 30 may further be configured to determine if the object detection and identification is indicative of that a none-UV-C reflective object is responsible for the differences between the reference picture and the probing picture, and if so, clear the alarm event. Hence, based on information from a camera 130 arranged in the space 1 it may be determined if the difference metric is indicative of harmful UV-C reflections or not.

The object detection function 30 may alternatively be configured to detect an object being responsible for the difference metric, i.e., an object possibly being responsible for reflecting UV-C light out from the area that is to be disinfected. With reference to Fig. 2, the object detection function 30 may identify the object 300 as being an object reflecting radar pulses and the possibly also UV-C light. The object detection function 30 may identify the object 300 as a laptop having an opened and angled cover. The cover is typically having metallic shielding and thereby there is a risk for UV-C light to reflect on the open laptop. Hence, an alarm event is triggered. However, in case the object detection function 30 identifies the object as an object without metal and conclude that there is no risk for UV-C reflections from the object. Hence, the one or more UV-C light sources 110 can safely be enabled. The object detection function 30 can be configured to issue such an instruction to the control function 26.

The object detection function 30 may also be used as a stand-alone indication on whether harmful UV-C reflections may be present or not upon activation of the one or more UV-C light sources 110. Hence, using object identification based on camera information it may be determined whether there are surfaces that may cause harmful UV-C light reflections upon activation of a UV-C light source 110. Hence, if the object identification indicates a risk for abnormal UV-C reflections, an alarm event may be triggered. The alarm event may also include which surface is indicated as causing the abnormal reflection.

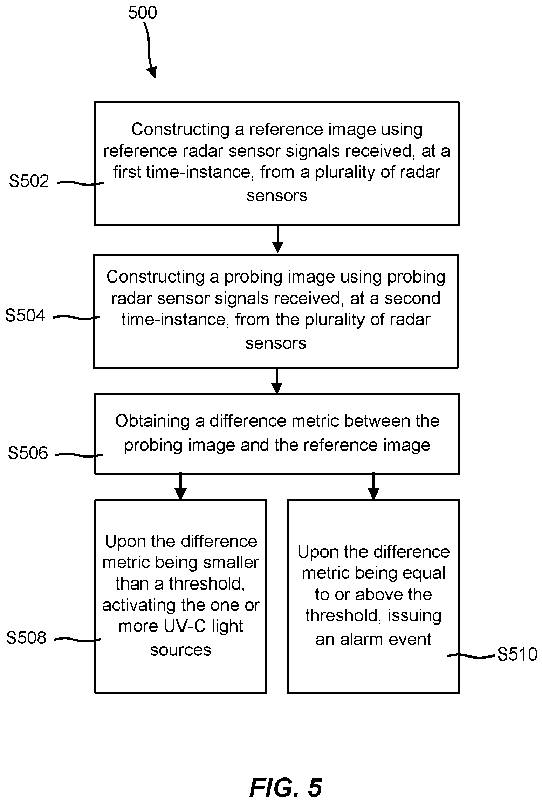

In connection with Fig. 5 a method 400 for controlling the one or more UV-C light sources 110 will be discussed Some of all the steps of the method 500 may be performed by the functions of the control engine 20 described above. However, it is equally realized that some or all of the steps of the method 500 may be performed by similar functions performed at other devices. The method 500 comprises the following steps. The steps may be performed in any suitable order.

Constructing S502 a reference image using reference radar sensor signals received, at a first time-instance, from the plurality of radar sensors 120. The construction of the reference image is discussed in greater detail above and reference is made to that discussion for details.

Constructing S504 a probing image using probing radar sensor signals received, at a second time-instance, from the plurality of radar sensors 120. At the second time-instance the one or more UV-C light sources 110 are inactive. The second time-instance is after the first time-instance. The construction of the probing image is discussed in greater detail above and reference is made to that discussion for details.

Obtaining S506 a difference metric between the probing image and the reference image. The difference metric is discussed in greater detail above and reference is made to that discussion for details. Upon the difference metric being smaller than a threshold, activating S508 the one or more UV-C light sources. Upon the difference metric being equal to or above the threshold, issuing S510 an alarm event.

The method may further comprise transforming a radar image constructed from radar sensor signals to an UV-C light reflection image. The transformation is dependent on a wavelength of EM-radiation emitted from the radar sensors and a wavelength of EM-radiation emitted from the one or more UV- C light sources. The reference image may be constructed as a reference UV-C light reflection image transformed from a reference radar image constructed from the reference radar sensor signals. The probing image may be constructed as a probing UV-C light reflection image transformed from a probing radar image constructed from the probing radar sensor signals.

The method may further comprise determining material properties of surfaces in the space from radar sensor signals received from the plurality of radar sensors. The transformation may be dependent on the material properties of the surfaces in the space.

The method may further comprise constructing a reference radar image from the reference radar sensor signals; activating the one or more UV-C light sources; constructing a reference UV-C light reflection image from reference UV-C sensor signals received, at the first time-instance, from one or more UV-C sensors 115 arranged in the space 1; and determining spatial correlation information from a spatial correlation between the reference UV-C light reflection image and the reference radar image. The probing image may be constructed from the probing radar sensor signals and the spatial correlation information.

The method may further comprise, at the first time-instance, acquiring a reference picture from a camera 130 arranged in the space 1; and at the second time-instance, acquiring a probing picture from the camera. The method may further comprise performing an object detection and identification based on differences between the reference picture and the probing picture; and determining if the object detection and identification is indicative of that a none-UV-C reflective object is responsible for the differences between the reference picture and the probing picture. If so, the method may further comprise clearing the alarm event.

The person skilled in the art realizes that the present invention by no means is limited to what is explicitly described above. On the contrary, many modifications and variations are possible within the scope of the appended claims.

For example, pictures from the one or more cameras 130 may be used for determining a material of the surfaces of the space 1. Possibly such determination may be made in combination with information from a radar image. This may be used for achieving an improved reference UV-C light reflection image. For example, a radar image may give a first indication of a material of a surface and object identification run on picture(s) captured by the one or more cameras 130 may confirm the material.

Further, 3D models of the space 1 may be used. A reference image may e.g., be created by simulating UV light emission according to the 3D model of the space 1. The 3D model may include furniture. UV-C reflections may be spatially correlated to radar reflections and hence a mapping between radar reflections and UV reflections can be made.

Additionally, variations can be understood and effected by the skilled person in practicing the claimed invention, from a study of the drawings, the disclosure, and the appended claims.

## Claims

1. A control engine (20) configured to control one or more UV-C light sources (110) configured to emit UV-C light in a space (1), the control engine (20) comprising:
circuitry (21) configured to execute:
a reference image acquiring function (24) configured to construct a reference image using reference radar sensor signals received, at a first time-instance, from a plurality of radar sensors (120) arranged in the space (1);
a probing radar acquiring function (25) configured to construct a probing image using probing radar sensor signals received, at a second time-instance at which the one or more UV-C light sources (110) are inactive, from the plurality of radar sensors (120), wherein the second time-instance is after the first time-instance; and
a control function (26) configured to obtain a difference metric between the probing image and the reference image,
wherein upon the difference metric being smaller than a threshold, the control function (26) is configured to activate the one or more UV-C light sources (110), or upon the difference metric being equal to or above the threshold, the control function (26) is configured to issue an alarm event.

2. The control engine according to claim 1,
wherein the circuitry (21) is further configured to execute a transformation function (27) configured to transform a radar image constructed from radar sensor signals to an UV-C light reflection image, wherein the transformation is dependent on a wavelength of EM-radiation emitted from the radar sensors and a wavelength of EM-radiation emitted from the one or more UV- C light sources,
wherein the reference image acquiring function (24) is configured to construct the reference image as a reference UV-C light reflection image transformed from a reference radar image constructed from the reference radar sensor signals, and
wherein probing image acquiring function (25) is configured to construct the probing image as a probing UV-C light reflection image transformed from a probing radar image constructed from the probing radar sensor signals.

3. The control engine according to claim 2, wherein the circuitry (21) is further configured to execute a material property determination function (28) configured to determine material properties of surfaces in the space from radar sensor signals received from the plurality of radar sensors arranged in the space,
wherein the transformation performed by the transformation function (27) is further dependent on the material properties of the surfaces in the space.

4. The control engine according to claim 1,
wherein the circuitry (21) is further configured to execute a correlation function (29) configured to:
construct a reference radar image from the reference radar sensor signals,
activate the one or more UV-C light sources,
construct a reference UV-C light reflection image from reference UV-C sensor signals received, at the first time-instance, from one or more UV-C sensors (115) arranged in the space (1), and
determine spatial correlation information from a spatial correlation between the reference UV-C light reflection image and the reference radar image;
wherein the probing image acquiring function (25) is configured to construct the probing image from the probing radar sensor signals and the spatial correlation information.

5. The control engine according to any one of claims 1-4, wherein the circuitry (21) is further configured to execute an object detection function (30) configured to:
at the first time-instance, acquire a reference picture from a camera (130) arranged in the space (1),
at the second time-instance, acquire a probing picture from the camera,
perform an object detection and identification based on differences between the reference picture and the probing picture, and
determine if the object detection and identification is indicative of that a none-UV-C reflective object is responsible for the differences between the reference picture and the probing picture and if so, clear the alarm event.

6. A UV-C light system (10) comprising:
one or more UV-C light sources (110) configured to emit UV-C light in a space (1);
a plurality of radar sensors (120) arranged in the space (1); and
a control engine (20) according to any one of claims 1-3.

7. The UV-C light system (10) according to claim 6, further comprising one or more UV-C sensors (115) arranged in the space (1), wherein the circuitry (21) of the control engine (20) is further configured to execute a correlation function (29) configured to:
construct a reference radar image from the reference radar sensor signals,
activate the one or more UV-C light sources,
construct a reference UV-C light reflection image from reference UV-C sensor signals received, at the first time-instance, from the one or more UV-C sensors (115) arranged in the space (1), and
determine spatial correlation information from a spatial correlation between the reference UV-C light reflection image and the reference radar image;
wherein the probing image acquiring function (25) is configured to construct the probing image from the probing radar sensor signals and the spatial correlation information.

8. The UV-C light system (10) according to claim 6 or 7, further comprising a camera (130) arranged in the space (1), wherein the circuitry (21) of the control engine (20) is further configured to execute an object detection function (30) configured to:
at the first time-instance, acquire a reference picture from the camera (130),
at the second time-instance, acquire a probing picture from the camera (130),
perform an object detection and identification based on differences between the reference picture and the probing picture, and
determine if the object detection and identification is indicative of that a none-UV-C reflective object is responsible for the differences between the reference picture and the probing picture and if so, clear the alarm event.

9. A method for controlling one or more UV-C light sources (110) configured to emit UV-C light in a space (1), the method comprising:
constructing a reference image using reference radar sensor signals received, at a first time-instance, from a plurality of radar sensors (120) arranged in the space (1);
constructing a probing image using probing radar sensor signals received, at a second time-instance at which the one or more UV-C light sources (110) are inactive, from the plurality of radar sensors (120), wherein the second time-instance is after the first time-instance;
obtaining a difference metric between the probing image and the reference image;
upon the difference metric being smaller than a threshold, activating the one or more UV-C light sources (110); and
upon the difference metric being equal to or above the threshold, issuing an alarm event.

10. The method of claim 9, further comprising:
transforming a radar image constructed from radar sensor signals to an UV-C light reflection image, wherein the transformation is dependent on a wavelength of EM-radiation emitted from the radar sensors and a wavelength of EM-radiation emitted from the one or more UV- C light sources,
wherein the reference image is constructed as a reference UV-C light reflection image transformed from a reference radar image constructed from the reference radar sensor signals, and
wherein the probing image is constructed as a probing UV-C light reflection image transformed from a probing radar image constructed from the probing radar sensor signals.

11. The method according to claim 10, further comprising determining material properties of surfaces in the space from radar sensor signals received from the plurality of radar sensors, wherein the transformation is further dependent on the material properties of the surfaces in the space.

12. The method according to claim 9, further comprising:
constructing a reference radar image from the reference radar sensor signals;
activating the one or more UV-C light sources (110);
constructing a reference UV-C light reflection image from reference UV-C sensor signals received, at the first time-instance, from one or more UV-C sensors (115) arranged in the space (1); and
determining spatial correlation information from a spatial correlation between the reference UV-C light reflection image and the reference radar image;
wherein the probing image is constructed from the probing radar sensor signals and the spatial correlation information.

13. The method according to claim 9, further comprising:
at the first time-instance, acquiring a reference picture from a camera (130) arranged in the space (1);
at the second time-instance, acquiring a probing picture from the camera;
performing an object detection and identification based on differences between the reference picture and the probing picture; and
determining if the object detection and identification is indicative of that a none-UV-C reflective object is responsible for the differences between the reference picture and the probing picture and if so, clearing the alarm event.

14. A non-transitory computer-readable storage medium having stored thereon instructions for implementing the method according to any one of claims 9-13, when executed on a device having processing capabilities.
